(19) Europäisches Patentamt
European Patent Office
Office européen des brevets

(11) EP 0 768 080 B1

(12) **FASCICULE DE BREVET EUROPEEN**

(45) Date de publication et mention de la délivrance du brevet:
**24.06.1998 Bulletin 1998/26**

(51) Int. Cl.$^6$: **A61K 7/36**

(21) Numéro de dépôt: **96401934.3**

(22) Date de dépôt: **10.09.1996**

# (54) Composition déodorante comprenant un sel hydrosoluble de zinc à titre d'agent absorbeur d'odeurs

Desodorisierende Zusammensetzung, die ein wasserlösliches Zinksalz als Geruchsabsorber enthält

Deodorant composition containing a hydrosoluble zinc salt as odour absorbing agent

(84) Etats contractants désignés:
**AT BE CH DE ES FR GB IT LI NL SE**

(30) Priorité: **16.10.1995 FR 9512098**

(43) Date de publication de la demande:
**16.04.1997 Bulletin 1997/16**

(73) Titulaire: **L'OREAL**
**75008 Paris (FR)**

(72) Inventeurs:
- **Ascione, Jean-Marc**
**75018 Paris (FR)**
- **Forestier, Serge**
**77410 Claye Souilly (FR)**
- **Rollat-Corvol, Isabelle**
**92100 Boulogne (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 216 558**
**FR-A- 2 398 495**
**US-A- 2 087 161**
**WO-A-93/01793**
**GB-A- 1 536 222**

EP 0 768 080 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne une composition déodorante comprenant au moins un sel hydrosoluble de zinc à titre d'agent absorbeur d'odeurs, en particulier une composition comprenant ledit sel de zinc comme seul agent absorbeur d'odeurs, et l'utilisation de ces compositions pour l'application topique humaine.

Elle concerne également un procédé de désodorisation mettant en oeuvre ladite composition et plus spécialement un procédé pour traiter les odeurs axillaires humaines consistant à appliquer sur la surface axillaire une quantité efficace de ladite composition.

Dans le domaine cosmétique, il est bien connu d'utiliser en application topique, des produits déodorants contenant des substances actives de type antitranspirant ou de type bactéricide pour diminuer voire supprimer les odeurs axillaires généralement désagréables.

Les substances antitranspirantes ont pour effet de limiter le flux sudoral. Elles sont généralement constituées de sels d'aluminium qui d'une part sont irritants pour la peau et qui d'autre part diminuent le flux sudoral en modifiant la physiologie cutanée, ce qui n'est pas satisfaisant.

Les substances bactéricides, en inhibant le développement de la flore cutanée responsable des odeurs axillaires, présentent l'inconvénient de n'être pas actifs sur l'odeur de la sueur déjà développée. Ces produits bactéricides, dont le plus couramment employé est le triclosan (5-chloro-2-(2,4-dichlorophénoxy) phénol), sont donc à long terme insuffisamment efficaces.

Dans le but d'obtenir une efficacité à plus long terme, on recherche des produits déodorants qui exercent une action d'absorbeur d'odeurs, c'est-à-dire des produits qui soient capables de capturer et retenir en leur sein les molécules responsables des mauvaises odeurs (cette définition est donnée dans l'ouvrage ⟨⟨ Cosmetic Science and Technology Series ⟩⟩ - 1988 / Volume 7 chap.10 - IIIc). Ces produits ont pour effet de diminuer plus ou moins fortement l'odeur de sueur déjà développée et ne présentent pas les inconvénients des substances actives antérieurement employées dans les compositions déodorantes.

Dans cette optique, certains sels de zinc ont déjà été proposés. On connaît notamment les sels de zinc de polyacides carboxyliques décrits dans le brevet américain 4 425 321 et en particulier ceux de l'acide dimerginique et ceux de l'acide trimerginique hydroxylé ou non hydroxylé.

Le document GB 1 536 222 décrit des compositions déodorantes comprenant un complexe soluble dans l'alcool.

Le document EP 0 216 558 décrit des compositions anti-microbiennes à base d'un sel de zinc soluble dans l'eau. Le sel de zinc est un chlorure, un acétate, un sulfate, un nitrate ou un phénylsulfonate. Les compositions obtenues son monophasées.

Le document US 2 087 161 décrit des compositions déodorantes comprenant du chlorure de zinc, de la cire et de l'alcool. La concentration en alcool est prédominante.

Le document FR 2 398 495 décrit des compositions déodorantes contenant un sel de polyacide carboxylique. Les compositions se présentent sous forme de lotion , de crème, de gel ou de poudre.

Le document WO 93/01793 décrit des compositions déodorantes à base de phénolsulfonate de zinc dans un support. Elles se présentent sous forme de solution ou de lotion ⟩⟩.

De tels absorbeurs d'odeurs à base de zinc sont encore néanmoins peu satisfaisants en raison du fait que leur insolubilité dans l'eau rend problématique leur formulation dans des compositions à base d'eau, ce qui est souvent le cas pour les formulations destinées, notamment, à un usage cosmétique.

Après de nombreuses recherches menées sur la question, la Demanderesse a maintenant découvert, de façon totalement inattendue et surprenante, que les sels de zinc hydrosolubles présentent également la propriété d'absorber les mauvaises odeurs, sans les inconvénients des substances actives antérieurement employées dans les compositions déodorantes, et avec l'avantage d'être hydrosolubles dans des proportions intéressantes et suffisantes pour être aisément formulables, notamment dans les compositions cosmétiques à base d'eau pour l'application topique humaine. Ces compositions sont en outre non-toxiques et non-irritantes.

Cette découverte est à la base de la présente invention.

La présente invention a ainsi pour objet une nouvelle composition déodorante comprenant à titre d'agent absorbeur d'odeurs, au moins un sel hydrosoluble de zinc.

Elle a également pour objet une composition déodorante comprenant comme unique agent absorbeur d'odeurs, un ou plusieurs sels hydrosolubles de zinc. Elle a aussi pour objet l'utilisation desdites compositions, plus spécialement dans des, ou pour la fabrication de, produits cosmétiques destinés à l'application topique humaine.

Elle a également pour objet l'utilisation d'au moins un sel hydrosoluble de zinc comme (de préférence unique) agent absorbeur d'odeurs dans, ou pour la fabrication de, une composition déodorante, en particulier cosmétique.

Elle a enfin pour objet un procédé de désodorisation mettant en oeuvre ladite composition, et plus particulièrement un procédé pour traiter les odeurs axillaires humaines, consistant à appliquer sur la surface axillaire une quantité efficace de ladite composition.

Au sens de la présente invention, on entend par sels hydrosolubles de zinc des sels de zinc d'acides organiques

ou minéraux dont la solubilité dans l'eau, exprimée en pourcentage pondéral d'ion Zn, est supérieure ou égale à 0,3. Des sels hydrosolubles de zinc particulièrement préférés selon la présente invention sont le pyrrolidone carboxylate de zinc (plus communément appelé pidolate de zinc), le sulfate de zinc, le chlorure de zinc, le lactate de zinc, le gluconate de zinc et le phénolsulfonate de zinc. Parmi les sels mentionnés ci-avant, on préfère encore plus particulièrement utiliser, selon la présente invention, le pyrrolidone carboxylate de zinc.

Dans les compositions déodorantes selon la présente invention, le sel hydrosoluble de zinc est généralement présent dans des proportions pondérales, calculées en ion Zn, allant environ de 0,01 à environ 10%, de préférence allant environ de 0,1 à environ 5%, et mieux encore allant environ de 0,1 à 2%, par rapport à l'ensemble de la composition.

Selon un mode préférentiel de réalisation des compositions selon l'invention, ces dernières ne contiennent pas, ou substantiellement pas, d'agents absorbeurs d'odeurs autres que des sels hydrosolubles de zinc.

Les compositions déodorantes de la présente invention sont formulées classiquement selon les applications auxquelles elles sont destinées.
Lorsqu'elles sont destinées à un usage cosmétique, elles sont plus particulièrement formulées dans un véhicule cosmétiquement acceptable qui peut être notamment essentiellement aqueux, et contenir des monoalcools en $C_1$-$C_4$ choisis de préférence parmi l'éthanol pour accélérer l'évaporation du produit, et/ou des polyols en $C_2$-$C_6$ choisis de préférence parmi le glycérol, le propylèneglycol ou le sorbitol, en tant qu'agents hydratants, dans une proportion pondérale allant de 0 à environ 50%. Elles peuvent également être formulées en émulsion eau-dans-huile, huile-dans-eau, ou en émulsion triple eau-dans-huile-dans-eau, (de telles émulsions sont connues et décrites par exemple par C. FOX dans ⟨⟨Cosmetics and Toiletries⟩⟩ - november 1986 - Vol 101 - pages 101-112), auquel cas le sel hydrosoluble de zinc est présent dans la phase aqueuse de l'émulsion. Selon un mode préférentiel de réalisation de la présente invention, les compositions sont formulées sous la forme d'une émulsion eau-dans-silicone, ce qui permet selon l'invention, de renforcer l'efficacité du sel hydrosoluble de zinc en tant qu'agent absorbeur d'odeurs, comparativement à une formulation aqueuse contenant une quantité équivalente du même sel hydrosoluble de zinc. Lesdites émulsions eau-dans-silicone comprennent alors : (i) une phase aqueuse dispersée et (ii) une phase grasse continue siliconée comprenant (a) une diméthicone volatile et (b) un agent émulsionnant siliconé.

(i) Phase aqueuse

La phase aqueuse de ces émulsions peut renfermer, en plus du sel hydrosoluble de zinc comme agent absorbeur d'odeurs, d'autres ingrédients tels que des humectants comme les polyols, des stabilisants comme l'acide citrique ou l'acide lactique, et éventuellement des monoalcools en $C_1$-$C_4$ tels que l'éthanol.

(ii) Phase grasse continue

Dans la phase grasse continue siliconée de ces émulsions,

- **(a)** ladite diméthicone volatile (point d'ébullition inférieur à 250°C) peut être linéaire ou cyclique; elle est plus particulièrement choisie dans le groupe formé par les méthylsiloxanes fluides comportant des unités de formule :

$$(CH_3)_a\ SiO_{(4-a)/2}$$

dans laquelle, a représente une valeur approximative allant de 2 à 3 (bornes incluses), et comprenant des unités siloxanes choisies dans le groupe formé par les unités suivantes : $(CH_3)_3\ SiO_{1/2}$, $(CH_3)_2\ SiO_{2/2}$, $CH_3\ SiO_{3/2}$, et $SiO_{4/2}$.
De préférence, le méthylsiloxane fluide volatil comprend essentiellement des unités diméthylsiloxane, et éventuellement des unités triméthylsiloxane. Des diméthicones volatiles plus particulièrement préférées sont les siloxanes cycliques de formule générale $[(CH_3)_2\ SiO]_x$ et les siloxanes linéaires de formule générale $(CH_3)_3\ SiO[(CH_3)_2\ SiO]_y\ Si(CH_3)_3$, et leurs mélanges, dans la formule desquelles x désigne un nombre entier allant de 3 à 6 et y désigne un nombre entier allant de 0 à 4.
Les diméthicones volatiles linéaires présentent généralement des viscosités inférieures à environ 5 centistokes à 25°C tandis que celles des diméthicones volatiles cycliques sont inférieures à environ 10 centistokes à 25°C.
Des exemples de telles diméthicones préférées comprennent par exemple (liste non limitative) : l'huile Silbione 70 045 V2 vendue par la société Rhône Poulenc, la DC 244 Fluid vendue par la société Dow Corning, qui sont des cyclotétradiméthylsiloxanes, la DC 245 Fluid vendue par la société Dow Corning, qui est un cyclopentadiméthylsiloxane, la Belsil DM 0 65 vendue par la société Wacker, qui est un hexaméthyldisiloxane.
La diméthicone volatile est présente dans l'émulsion eau-dans-silicone selon l'invention dans des proportions pondérales allant d'environ 1 à environ 25%, de préférence allant d'environ 1 à environ 15%, par rapport à l'ensemble de l'émulsion,

- **(b)** l'agent émulsionnant siliconé est plus particulièrement choisi dans le groupe formé par les polydiorganosiloxanes de formules (I) et (II) suivantes, ces composés pouvant eux-mêmes être dispersés dans une diméthicone volatile,

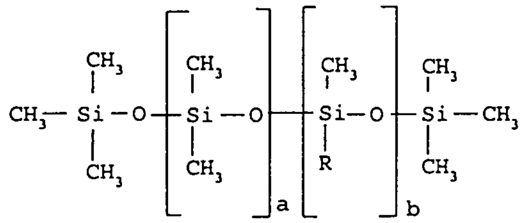

(I)

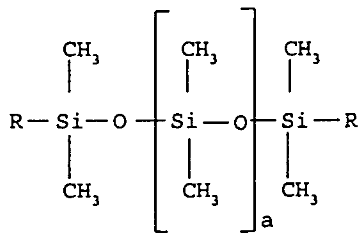

(II)

dans lesquelles,

R désigne le groupement :

$$-C_nH_{2n}-(OC_2H_4)_x-(OC_3H_6)_y-O-R',$$

R' désigne un atome d'hydrogène ou un radical alkyle linéaire ou ramifié comportant de 1 à environ 12 atomes de carbone,
a et b désignent respectivement un nombre entier allant de 1 à environ 500,
n est un nombre entier allant de 2 à 12,
x et y désignent respectivement un nombre entier allant de 0 à environ 50,
la somme x+y étant supérieure ou égale à la valeur 1.

De préférence, le polydiorganosiloxane émulsionnant est un polymère de formule (I) dans laquelle, a est un nombre entier allant de 2 à 450, b est un nombre entier allant de 2 à 40, n est un nombre entier allant de 2 à 5, x est un nombre entier allant de 1 à 30, y est un nombre entier allant de 0 à 30, avec $x \geq y$.

Avantageusement, selon l'invention, le polydiorganosiloxane émulsionnant se présente en dispersion dans une diméthicone volatile, la dispersion comprenant, par exemple en volume, de 1 à 20% de polydiorganosiloxane et de 80 à 99% de diméthicone volatile. Une dispersion particulièrement préférée comprend, en volume, 10% de polydiorganosiloxane dans la diméthicone volatile. Ainsi, un émulsionnant siliconé plus particulièrement préféré selon l'invention est un mélange de cyclométhicone et de diméthicone copolyol (nomenclatures C.T.F.A. - 4ème édition - 1991) vendu par la société Dow Corning sous la dénomination commerciale Silicone DC 3225 C.

Dans les émulsions eau-dans-silicone préférées selon l'invention, le polydiorganosiloxane émulsionnant de formule (I) ou (II) est généralement présent dans des proportions pondérales allant d'environ 0,1 à environ 20%, de préférence allant d'environ 0,5 à environ 15%, la phase aqueuse représente une quantité pondérale allant environ de 15 à environ 90%, et la phase grasse continue siliconée, environ de 10 à environ 85% du poids total de l'émulsion.

Des émulsions transparentes de ce type peuvent être obtenues lorsque l'indice de réfraction (ir) de la phase aqueuse est voisin de l'indice de réfraction de la phase grasse continue siliconée :

$$\Delta ir = (\text{ ir phase grasse siliconée - ir phase aqueuse }) < 0{,}01.$$

Lorsque les compositions déodorantes selon l'invention sont destinées à l'usage cosmétique, elles peuvent se présenter sous forme de lotions, de crèmes ou de gels fluides distribués en spray aérosol, en flacon pompe ou en roll-on, sous forme de crèmes épaisses distribuées en tubes et sous forme de sticks, et contenir à cet égard les ingrédients et propulseurs généralement utilisés dans ce type de produits et bien connus de l'homme de l'art, sous réserve qu'ils n'interfèrent pas avec le sel hydrosoluble de zinc décrit dans la présente invention.

L'invention peut aussi trouver des applications intéressantes dans le domaine des produits d'entretien et désodorisants divers (air ambiant, textiles, réfrigérateurs, vide-ordures, poubelles, litières et cages d'animaux domestiques ou gaines de ventilation des immeubles).

Des exemples concrets, mais nullement limitatifs, illustrant l'invention vont maintenant être donnés.

EXEMPLE 1

On a réalisé un test d'absorption d'odeurs sur de la sueur naturelle.
Au préalable, on s'est assuré que ce test était réellement un test d'absorption d'odeurs en vérifiant qu'un échantillon renfermant un bactéricide tel que le triclosan n'avait aucun effet d'absorption sur l'odeur de sueur.
On a donc prélevé en sauna les sueurs axillaires de plusieurs modèles humains que l'on a réunies pour réaliser un échantillon de sueur. Pour obtenir l'odeur nauséabonde caractéristique de la sueur, avant d'introduire la substance active, on a incubé cet échantillon de sueur pendant 14 heures à la température de 37°C. Dans chaque flacon de substance active à tester ainsi que dans un flacon témoin (sans substance active), on a introduit 1 ml de l'échantillon de sueur incubée. Puis on a introduit la substance active : 5,5 mg d'ion Zn sous forme de pidolate de zinc, de chlorure de zinc, de gluconate de zinc, de lactate de zinc, de phénolsulfonate de zinc et de sulfate de zinc, respectivement dans chacun des flacons de substance active à tester.
On a ensuite procédé à une deuxième incubation des flacons pendant 24 heures à la température de 37°C, puis à une troisième incubation pendant 3 jours à température ambiante.
Huit juges ont ensuite senti (〈〈sniff-test 〉〉) chacun de ces flacons et noté l'intensité de l'odeur sur une échelle de 0 à 4.

Note 0 = pas d'odeur.
Note 4 = odeur forte.

Résultats: le flacon témoin sans sel hydrosoluble de zinc a été noté 4, tandis que les flacons contenant les sels hydrosolubles de zinc mentionnés ci-avant ont été notés de 1,25 à 1,68, les meilleurs résultats ayant été obtenus avec le pidolate de zinc.

Ces résultats démontrent que les sels hydrosolubles de zinc présentent une importante capacité d'absorption des odeurs de la sueur naturelle.

EXEMPLE 2

On a réalisé les deux formules suivantes (une lotion aqueuse et une émulsion eau-dans-silicone) :

Lotion aqueuse :

| | |
|---|---|
| Pidolate de zinc | 3 g |
| Eau déminéralisée | qsp 100 g |

Emulsion eau-dans-silicone :

| | | |
|---|---|---|
| **A**/ Phase grasse : | | |
| Huile Silbione 70 045 V2. | | 6,6 g |
| Silicone DC 3225 C | | 9,4 g |
| **B**/ Phase aqueuse : | | |
| Pidolate de zinc | | 3 g |
| Alcool éthylique | | 11,1 g |
| Propylèneglycol | | 38,7 g |
| Eau déminéralisée | qsp | 100 g |

On a versé lentement la phase B dans la phase A, sous une agitation mécanique douce et à température ambiante. On a ensuite encore agité pendant 5 minutes tout en augmentant progressivement la vitesse d'agitation.
Puis on a mesuré in vivo l'efficacité déodorante (par absorption de l'odeur de la sueur naturelle) de ces deux formules, contenant chacune la même quantité de pidolate de zinc.
Sur 20 modèles humains préalablement conditionnés (c'est-à-dire n'ayant pas reçu d'application de déodorant bactéricide ou de déodorant antitranspirant pendant une semaine), on a appliqué d'une part la lotion aqueuse et d'autre part l'émulsion décrites ci-avant sous une aisselle, l'autre aisselle servant de témoin. Dix juges ont ensuite effectué le 《sniff test》 et noté l'intensité de l'odeur suivant une échelle de 0 à 9.

Note 0 = pas d'odeur.
Note 9 = odeur extrêmement puissante.

Les résultats, exprimés par la différence [moyenne des notes des aisselles témoins] - [moyennes des notes des aisselles traitées], ont été les suivants :

lotion aqueuse = 0,5
émulsion eau-dans-silicone = 1,1.

Ils démontrent que la formule en émulsion présente une efficacité déodorante par absorption des odeurs de la sueur naturelle supérieure à celle de la lotion aqueuse.

EXEMPLE 3

On a réalisé un gel déodorant transparent de composition suivante :

Emulsion eau-dans-silicone :

| | | |
|---|---|---|
| **A**/ Phase grasse : | | |
| Silicone DC 244 Fluid | | 6,5 g |
| Silicone DC 3225 C | | 9,4 g |
| **B**/ Phase aqueuse : | | |
| Sulfate de zinc ($ZnSO_4$, $_7H_2O$) | | 4,1 g |
| Glycérine | | 37 g |
| Eau déminéralisée | qsp | 100 g |

Ce gel cosmétique est efficace pour absorber les odeurs axillaires humaines.

## Revendications

1. Composition déodorante, caractérisée par le fait qu'elle comprend dans un support du type émulsion eau-dans-silicone, au moins un sel de zinc d'acide organique ou minéral, dont la solubilité dans l'eau est d'au moins 0,3% en poids, exprimée en ion Zn.

2. Composition selon la revendication 1, caractérisée par le fait qu'elle est exempte d'agent absorbeur d'odeurs autre qu'un sel hydrosoluble de zinc.

3. Composition selon la revendication 1, caractérisée par le fait qu'il s'agit d'un pyrrolidone carboxylate de zinc.

4. Composition selon la revendication 1, caractérisée par le fait qu'il s'agit d'un sulfate de zinc, d'un chlorure de zinc, d'un lactate de zinc, d'un gluconate de zinc ou d'un phénolsulfonate de zinc.

5. Composition selon l'une quelconque des revendications précédentes, caractérisée par le fait que le sel hydrosoluble de zinc est présent dans des concentrations pondérales en ion Zn allant de 0,01 à 10% par rapport au poids total de la composition.

6. Composition selon la revendication 5, caractérisée par le fait que le sel hydrosoluble de zinc est présent dans des concentrations pondérales en ion Zn allant de 0,1 à 5%, et de préférence allant de 0,1 à 2% par rapport au poids total de la composition.

7. Composition selon la revendication 1, caractérisée par le fait que ladite émulsion est essentiellement constituée d'une phase aqueuse dispersée comprenant le sel hydrosoluble de zinc et d'une phase grasse continue siliconée comprenant (i) une diméthicone volatile et (ii) un agent émulsionnant siliconé, éventuellement dispersé dans une diméthicone volatile, et choisi parmi les polydiorganosiloxanes de formules (I) et (II) suivantes :

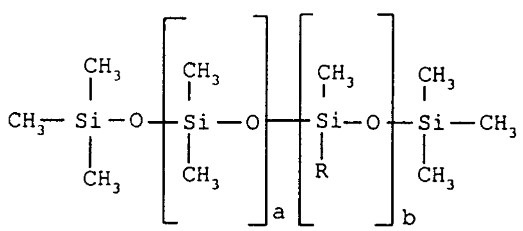

(I)

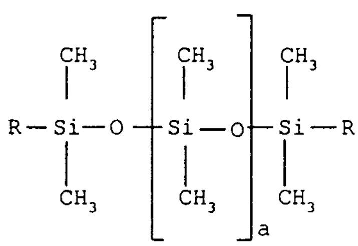

(II)

dans lesquelles,

R désigne le groupement :$--C_nH_{2n}--(OC_2H_4-)_x--(-OC_3H_6-)_y--O--R'$,
R' désigne un atome d'hydrogène ou un radical alkyle linéaire ou ramifié comportant de 1 à environ 12 atomes de carbone,
a et b désignent respectivement un nombre entier allant de 1 à environ 500,
n est un nombre entier allant de 2 à 12,
x et y désignent respectivement un nombre entier allant de 0 à environ 50,
la somme x+y étant supérieure ou égale à la valeur 1.

8. Composition selon la revendication 7, caractérisée par le fait que l'agent émulsionnant siliconé est un mélange de cyclométhicone et de diméthicone copolyol.

9. Composition selon la revendication 7 ou 8, caractérisé par le fait que le polydiorganosiloxane émulsionnant de formule (I) ou (II) est présent dans des concentrations pondérales allant de 0,1 à 20%, la phase aqueuse représente une quantité pondérale allant de 15 à 90% et la phase grasse siliconée représente une quantité pondérale allant de 10 à 85 % par rapport au poids total de la composition.

10. Composition déodorante, caractérisée par le fait qu'elle comprend, dans un support convenable, un pyrrolidone carboxylate de zinc.

11. Composition selon la revendication 10, caractérisée par le fait qu'elle est exempte d'agent absorbeur d'odeurs autre que le pyrrolidone carboxylate de zinc.

12. Composition selon l'une quelconque des revendications 10 ou 11, caractérisée par la fait que le pyrrolidone carboxylate de zinc est présent dans des concentrations pondérales en ion Zn allant de 0,01 à 10 % par rapport au poids total de la composition.

13. Composition selon la revendication 12 caractérisée par le fait que le sel hydrosoluble de zinc est présent dans des concentrations pondérales en ion Zn allant de 0,1 à 5%, et de préférence allant de 0,1 à 2 % par rapport au poids total de la composition.

14. Composition selon l'une quelconque des revendications 10 à 13 caractérisée par le fait que ledit support est constitué d'une phase aqueuse ou d'une émulsion.

15. Utilisation d'une composition telle que définie à l'une quelconque des revendications 1 à 14, dans des, ou pour la fabrication de, produits cosmétiques destinés à l'application topique humaine, en particulier des déodorants.

16. Utilisation d'au moins un sel de zinc d'acide organique ou minéral, dont la solubilité est d'au moins 0,3% en poids, exprimée en ion Zn dans une émulsion eau-dans-silicone, comme agent absorbeur d'odeurs dans, ou pour la fabrication de, une composition déodorante, en particulier cosmétique.

17. Utilisation d'au moins un sel de zinc d'acide organique ou minéral, dont la solubilité est d'au moins 0,3% en poids, exprimée en ion Zn dans une émulsion eau-dans-silicone, comme unique agent absorbeur d'odeurs dans, ou pour la fabrication de, une composition déodorante, en particulier cosmétique.

18. Utilisation du pyrrolidone carboxylate de zinc, comme agent absorbeur d'odeurs dans, ou pour la fabrication de, une composition déodorante, en particulier cosmétique.

19. Utilisation du pyrrolidone carboxylate de zinc, comme unique agent absorbeur d'odeurs dans, ou pour la fabrication de, une composition déodorante, en particulier cosmétique.

20. Procédé pour traiter les odeurs axillaires humaines, caractérisé par le fait qu'il consiste à appliquer une quantité efficace d'une composition telle que définie à l'une quelconque des revendications 1 à 14 sur la surface axillaire humaine.

**Claims**

1. Deodorant composition, characterized in that it comprises, in a vehicle of the water-in-silicone emulsion type, at least one zinc salt of an organic or inorganic acid, the solubility of which in water is at least 0.3% by weight, expressed as Zn ion.

2. Composition according to Claim 1, characterized in that it is free from odour-absorbing agent other than a water-soluble zinc salt.

3. Composition according to Claim 1, characterized in that it relates to a zinc pyrrolidonecarboxylate.

4. Composition according to Claim 1, characterized in that it relates to a zinc sulphate, a zinc chloride, a zinc lactate,

a zinc gluconate or a zinc phenolsulphonate.

5. Composition according to any one of the preceding claims, characterized in that the water-soluble zinc salt is present in concentrations by weight as Zn ion ranging from 0.01 to 10% with respect to the total weight of the composition.

6. Composition according to Claim 5, characterized in that the water-soluble zinc salt is present in concentrations by weight as Zn ion ranging from 0.1 to 5% and preferably ranging from 0.1 to 2% with respect to the total weight of the composition.

7. Composition according to Claim 1, characterized in that the said emulsion is essentially composed of a dispersed aqueous phase comprising the water-soluble zinc salt and of a continuous silicone fatty phase comprising (i) a volatile dimethicone and (ii) a silicone emulsifying agent, optionally dispersed in a volatile dimethicone, chosen from the polydiorganosiloxanes of following formulae (I) and (II):

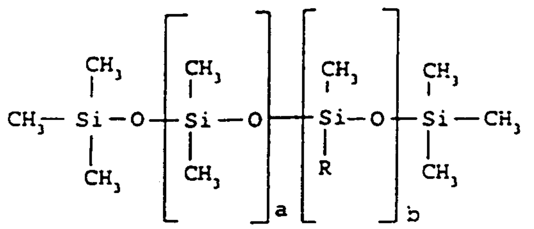

(I)

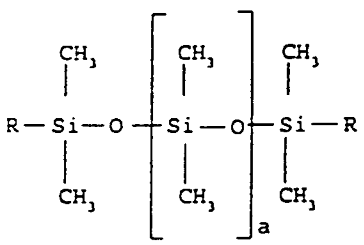

(II)

in which,

R denotes the group: $--C_nH_{2n}--(-OC_2H_4-)_x--(OC_3H_6-)_y--O--R'$,
R' denotes a hydrogen atom or a linear or branched alkyl radical containing from 1 to approximately 12 carbon atoms,
a and b respectively denote an integer ranging from 1 to approximately 500,
n is an integer ranging from 2 to 12,
x and y respectively denote an integer ranging from 0 to approximately 50, the sum x+y being greater than or equal to the value 1.

8. Composition according to Claim 7, characterized in that the silicone emulsifying agent is a mixture of cyclomethicone and of dimethicone copolyol.

9. Composition according to Claim 7 or 8, characterized in that the emulsifying polydiorganosiloxane of formula (I) or (II) is present in concentrations by weight ranging from 0.1 to 20%, the aqueous phase represents an amount by weight ranging from 15 to 90% and the silicone fatty phase represents an amount by weight ranging from 10 to 85% with respect to the total weight of the composition.

10. Deodorant composition, characterized in that it comprises, in a suitable vehicle, a zinc pyrrolidonecarboxylate.

11. Composition according to Claim 10, characterized in that it is free from odour-absorbing agent other than zinc pyrrolidonecarboxylate.

12. Composition according to either one of Claims 10 and 11, characterized in that zinc pyrrolidonecarboxylate is present in concentrations by weight as Zn ion ranging from 0.01 to 10% with respect to the total weight of the composition.

13. Composition according to Claim 12, characterized in that the water-soluble zinc salt is present in concentrations by weight as Zn ion ranging from 0.1 to 5% and preferably ranging from 0.1 to 2% with respect to the total weight of the composition.

14. Composition according to any one of Claims 10 to 13, characterized in that the said vehicle is composed of an aqueous phase or of an emulsion.

15. Use of a composition as defined in any one of Claims 1 to 14 in, or for the manufacture of, cosmetic products intended for human topical application, in particular deodorants.

16. Use of at least one zinc salt of an organic or inorganic acid, the solubility of which is at least 0.3% by weight, expressed as Zn ion, in a water-in-silicone emulsion, as odour-absorbing agent in, or for the manufacture of, a deodorant composition, in particular a cosmetic composition.

17. Use of at least one zinc salt of an organic or inorganic acid, the solubility of which is at least 0.3% by weight, expressed as Zn ion, in a water-in-silicone emulsion, as sole odour-absorbing agent in, or for the manufacture of, a deodorant composition, in particular a cosmetic composition.

18. Use of zinc pyrrolidonecarboxylate as odour-absorbing agent in, or for the manufacture of, a deodorant composition, in particular a cosmetic composition.

19. Use of zinc pyrrolidonecarboxylate as sole odour-absorbing agent in, or for the manufacture of, a deodorant composition, in particular a cosmetic composition.

20. Process for treating human axillary smells, characterized in that it comprises the application of an effective amount of a composition as defined in any one of Claims 1 to 14 on the human axillary surface.

**Patentansprüche**

1. Desodorisierende Zusammensetzungen,
dadurch **gekennzeichnet,** daß
sie in einem Träger vom Typ Wasser-in-Silicon-Emulsion mindestens ein Zinksalz einer organischen oder anorganischen Säure enthalten, dessen Löslichkeit in Wasser mindestens 0,3 Gew.-%, bezogen auf das Zinkion, beträgt.

2. Zusammensetzungen nach Anspruch 1, dadurch **gekennzeichnet,** daß sie neben dem wasserlöslichen Zinksalz keinen weiteren Geruchsabsorber enthalten.

3. Zusammensetzungen nach Anspruch 1, dadurch **gekennzeichnet,** daß es sich um eine Zinkpyrrolidoncarboxylat handelt.

4. Zusammensetzungen nach Anspruch 1, dadurch **gekennzeichnet,** daß es sich um ein Zinksulfat, Zinkchlorid, Zinklactat, Zinkgluconat oder Zinkphenolsulfonat handelt.

5. Zusammensetzungen nach einem der vorhergehenden Ansprüche, dadurch **gekennzeichnet,** daß das wasserlösliche Zinksalz in Gewichtsanteilen, die bezogen auf das Zinkion berechnet sind, von 0,01 bis 10 %, bezogen auf das Gesamtgewicht der Zusammensetzung, vorliegt.

6. Zusammensetzungen nach Anspruch 5, dadurch **gekennzeichnet,** daß das wasserlösliche Zinksalz in bezogen auf das Zinkion berechneten Gewichtsanteilen von 0,1 bis 5 % und vorzugsweise von 0,1 bis 2 %, bezogen auf das Gesamtgewicht der Zusammensetzung, vorliegt.

7. Zusammensetzungen nach Anspruch 1, dadurch **gekennzeichnet,** daß die Emulsion im wesentlichen aus einer dispergierten wäßrigen Phase, die das wasserlösliche Zinksalz enthält, und einer kontinuierlichen Silicon-Fettphase besteht, die (i) ein flüchtiges Dimethicon und (ii) einen Silicon-Emulgator enthält, welcher gegebenenfalls in

einem flüchtigen Dimethicon dispergiert ist und unter den Polydiorganosiloxanen der folgenden Formeln (I) und (II) ausgewählt ist:

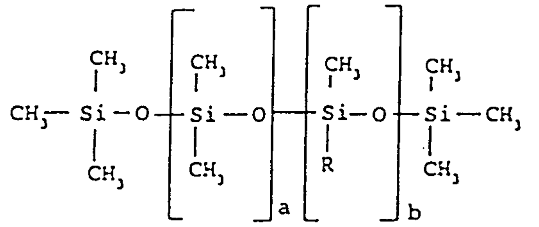

(I)

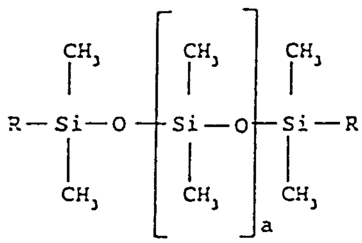

(II)

worin bedeuten:

R die Gruppe:

$$-C_nH_{2n}-(OC_2H_4)_x-(OC_3H_6)_y-O-R',$$

R' Wasserstoff oder eine geradkettige oder verzweigte Alkylgruppe mit 1 bis etwa 12 Kohlenstoffatomen,
a bzw. b eine ganze Zahl im Bereich von 1 bis etwa 500,
n eine ganze Zahl von 2 bis 12,
x bzw. y Null oder eine ganze Zahl von 1 bis etwa 50, wobei die Summe x+y mindestens 1 ist.

8. Zusammensetzungen nach Anspruch 7, dadurch **gekennzeichnet,** daß der Silicon-Emulgator ein Gemisch von Cyclomethicon und Dimethiconcopolyol ist.

9. Zusammensetzungen nach Anspruch 7 oder 8, dadurch **gekennzeichnet,** daß das emulgierende Polydiorganosiloxan der Formel (I) oder (II) in Gewichtsanteilen von 0,1 bis 20 % vorliegt, die wäßrige Phase einen Gewichtsanteil von 15 bis 90 % und die Silicon-Fettphase einen Gewichtsanteil von 10 bis 85 %, bezogen auf das Gesamtgewicht der Zusammensetzung, ausmacht.

10. Desodorisierende Zusammensetzungen, dadurch **gekennzeichnet,** daß sie in einem geeigneten Träger ein Zinkpyrrolidoncarboxylat enthalten.

11. Zusammensetzungen nach Anspruch 10, dadurch **gekennzeichnet,** daß sie neben dem Zinkpyrrolidoncarboxylat keinen Geruchsabsorber enthalten.

12. Zusammensetzungen nach einem der Ansprüche 10 oder 11, dadurch **gekennzeichnet,** daß das Zinkpyrrolidoncarboxylat in auf das Zinkion bezogenen Gewichtsanteilen von 0,01 bis 10 %, bezogen auf das Gesamtgewicht der Zusammensetzung, vorliegt.

13. Zusammensetzungen nach Anspruch 12, dadurch **gekennzeichnet,** daß das wasserlösliche Zinksalz in Gewichtsanteilen, die bezogen auf das Zinkion berechnet sind, von 0,1 bis 5 % und vorzugsweise von 0,1 bis 2 %, bezogen auf das Gesamtgewicht der Zusammensetzung, vorliegt.

14. Zusammensetzungen nach einem der Ansprüche 10 bis 13, dadurch **gekennzeichnet,** daß der Träger aus einer wäßrigen Phase oder einer Emulsion besteht.

15. Verwendung einer Zusammensetzung nach einem der Ansprüche 1 bis 14 in kosmetischen Produkten, die zur topischen Anwendung beim Menschen bestimmt sind, insbesondere in Deodorants, oder zur Herstellung dieser Produkte.

16. Verwendung mindestens eines Zinksalzes einer organischen oder anorganischen Säure, dessen Löslichkeit, bezogen auf das Zinkion, mindestens 0,3 Gew.-% beträgt, in einer Wasser-in-Silicon-Emulsion als Geruchsabsorber in desodorisierenden, insbesondere kosmetischen Zusammensetzungen oder zu deren Herstellung.

17. Verwendung mindestens eines Zinksalzes einer organischen oder anorganischen Säure, dessen Löslichkeit, bezogen auf das Zinkion in einer Wasser-in-Silicon-Emulsion, mindestens 0,3 Gew.-% beträgt, als einziger Geruchsabsorber in desodorisierenden, insbesondere kosmetischen Zusammensetzungen oder zu deren Herstellung.

18. Verwendung von Zinkpyrrolidoncarboxylat als Geruchsabsorber in desodorisierenden insbesondere kosmetischen Zusammensetzungen oder zu deren Herstellung.

19. Verwendung von Zinkpyrrolidoncarboxylat als einzigen Geruchsabsorber in desodorisierenden, insbesondere kosmetischen Zusammensetzungen oder zu deren Herstellung.

20. Verfahren zur Behandlung von menschlichem Achselgeruch, dadurch **gekennzeichnet,** daß es darin besteht, eine wirksame Menge einer Zusammensetzung nach einem der Ansprüche 1 bis 14 auf die Oberfläche der menschlichen Achselhöhle aufzutragen.